(19) Europäisches Patentamt / European Patent Office / Office européen des brevets

(11) **EP 2 545 951 B1**

(12) **FASCICULE DE BREVET EUROPEEN**

(45) Date de publication et mention
de la délivrance du brevet:
**07.04.2021   Bulletin 2021/14**

(51) Int Cl.:
***A61M 16/00*** *(2006.01)*

(21) Numéro de dépôt: **11005677.7**

(22) Date de dépôt: **12.07.2011**

(54) **APPAREIL DE TRAITEMENT DE TROUBLES RESPIRATOIRES DU SOMMEIL**

VORRICHTUNG ZUR BEHANDLUNG VON SCHLAFATMUNGSSTÖRUNGEN

DEVICE FOR TREATING SLEEP DISORDERED BREATHING

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(43) Date de publication de la demande:
**16.01.2013   Bulletin 2013/03**

(73) Titulaire: **SEFAM
92200 Neuilly sur Seine (FR)**

(72) Inventeurs:
• **Mougel, Laurent
88100 Sainte Marguerite (FR)**
• **Nicolazzi, Pascal
54480 Gondreville (FR)**

(74) Mandataire: **Novagraaf Technologies
Bâtiment O2
2, rue Sarah Bernhardt
CS90017
92665 Asnières-sur-Seine Cedex (FR)**

(56) Documents cités:
**WO-A1-2005/070488     WO-A2-2004/112680
WO-A2-2008/100859     US-A1- 2003 121 519
US-A1- 2008 097 234     US-A1- 2009 241 952**

EP 2 545 951 B1

**Description**

La présente invention concerne un appareil générateur de débit de gaz délivrant une pression positive utilisable pour le traitement des troubles respiratoires du sommeil.

Traditionnellement, un patient souffrant de troubles respiratoires du sommeil est traité grâce à un appareil de pression positive qui délivre au patient un flux de gaz à une pression de traitement prédéterminée.

On entend par « pression positive » une ventilation mécanique permettant de traiter, par exemple, certaines maladies comme le syndrome d'apnée du sommeil.

Cette pression de traitement pourrait procurer chez certains patients un sentiment d'inconfort dû essentiellement à une sensation de difficulté à l'expiration, surtout lors des modes de traitement durant lesquels une pression de traitement constante est délivrée pendant la phase expiratoire.

Si la valeur de la pression de traitement est amoindrie de manière continue de sorte à ne plus procurer une gêne au patient, alors le traitement n'est plus efficace.

L'objectif est de proposer une fonction permettant de diminuer ce sentiment d'inconfort lors du traitement (qu'il soit CPAP, Bi-level ou auto-CPAP) tout en préservant son efficacité.

Les termes CPAP, Bi-level et auto-CPAP sont traditionnellement employés dans la technologie considérée.

On entend par CPAP, un traitement lors duquel la pression délivrée au patient reste constante à une valeur prédéterminée (Pcpap) au cours de la respiration du patient, c'est-à-dire pendant les phases inspiratoires et expiratoires.

On entend par Bi-level, un traitement lors duquel la pression délivrée au patient varie selon les phases d'inspiration ou d'expiration. Lors d'une phase d'inspiration du patient, la pression délivrée est alors constante et égale à une valeur de pression (Pinsp), et lors d'une phase d'expiration, la pression délivrée est constante et égale à une valeur de pression (Pexp), (Pexp) étant plus faible que (Pinsp). Les valeurs des pressions (Pexp) et (Pinsp), constantes, sont généralement prédéterminées lors de l'établissement du traitement, par exemple par le praticien ayant prescrit le traitement.

On entend par auto-CPAP un traitement lors duquel la pression de traitement délivrée au patient est continue au cours de la respiration (quelle que soit la phase respiratoire, i.e. inspiration ou expiration) et dont le niveau s'adapte en fonction des événements respiratoires détectés chez le patient.

On connait par exemple des appareils qui, afin de maintenir une pression stable au niveau des voies aériennes du patient, doivent compenser les variations de pressions inhérentes aux débits de respiration du patient et aux débits de fuites au travers des résistances pneumatiques de l'ensemble du circuit d'air de l'appareil.

Pour ce faire certains appareils disposent d'un dispositif de régulation de la pression au niveau d'un masque (Pmask) porté par le patient fonctionnant par exemple sur le principe suivant :

- Soit un capteur de pression permet de mesurer la pression à la sortie de l'appareil (Pout), et de communiquer la mesure de pression (Pout) associée à l'appareil ;

- Soit un capteur de débit permet de mesurer le débit total du flux d'air sortant de l'appareil (Qt) et de communiquer la mesure à l'appareil ;

- Soit une ou plusieurs tables de valeurs sont mémorisées dans la mémoire de l'appareil, ces tables contenant des valeurs de compensation de la pression pour chaque débit de l'appareil, ces tables de valeurs peuvent être déterminées empiriquement pour une ou plusieurs configurations pneumatiques ;

- Soit une ou plusieurs équations permettent de déterminer la compensation de pression à délivrer en fonction du débit de sortie de l'appareil ;

- Soit une ou plusieurs tables de valeurs mémorisées contenant la valeur des coefficients déterminés empiriquement permettent la détermination de la compensation de pression par approximation linéaire pour différentes plages de débit de sortie de l'appareil, et cela pour une ou plusieurs configurations pneumatiques.

Soit, selon un autre exemple : la compensation des variations de pressions inhérentes aux débits de respiration du patient et aux débits de fuite au travers des résistances pneumatiques de l'ensemble du circuit d'air de l'appareil peut fonctionner de la façon suivante:

$$(Pmask \; estimée) = (Pout) - (Ai * (Qt) + Bi),$$

où (Pmask estimée) est la pression estimée au niveau du masque du patient, (Pout) est la pression mesurée en sortie de l'appareil, (Qt) est le débit total du flux d'air sortant de l'appareil, et (Ai) et (Bi) sont des valeurs de coefficients déterminés empiriquement permettant la détermination de la compensation de pression par approximation linéaire pour différentes plages de débit total (Qt) en sortie de l'appareil. Ce type de procédé de régulation a été déjà expliqué dans le brevet américain US2009/0241952 A1 de Nicolazzi et al.

Dans ce contexte, « masque » désigne toute interface portée par le patient à proximité de ses voies respiratoires, par exemple un masque nasal, un masque bucco-nasal, ou un masque narinaire.

Cependant, ces principes de compensation des variations de pression inhérentes aux résistances pneumatiques du circuit ne permettent pas de surcompenser ou sous-compenser la pression délivrée au patient de manière à diminuer la pression de traitement lors de l'expiration et de l'augmenter lors de l'inspiration, et le sentiment d'inconfort ou de gêne persiste

Les documents WO 2008/100859 A2, WO 2004/112680 A2, US 2003/121519 A1, WO 2005/070488 A1 décrivent des appareils de traitement des troubles respiratoires du sommeil d'un patient connus de l'art antérieur.

La présente invention concerne un appareil de traitement des troubles respiratoires du sommeil d'un patient tel que défini dans la revendication 1.

Le problème technique que l'on cherche à résoudre par la présente invention est :

- d'une part, de pouvoir garder une valeur de pression au masque (Pmask) la plus juste possible, c'est-à-dire la plus proche possible de la valeur de consigne, fixée par le praticien par exemple, ce qui est nécessaire à l'efficacité du traitement pour le patient, plus particulièrement à la fin de la phase expiratoire pour ne pas dégrader l'efficacité du traitement ;
- et d'autre part, de pouvoir surcompenser ou sous-compenser la pression délivrée au patient pour améliorer son confort.

A cet effet est proposé, suivant l'invention, un appareil de traitement des troubles respiratoires du sommeil d'un patient tel que défini dans la revendication 1. L'appareil selon l'invention permet de mettre en oeuvre un procédé, dans lequel, à chaque instant :

- On mesure le débit total (Qt) d'air délivré par l'appareil ;
- On calcule la différence (Dq) entre le débit total (Qt) et une valeur de base du débit total (Qb) correspondant aux valeurs de débits de fuites existantes dans le circuit patient ;
- On détermine un indice (i) de débit respiratoire à partir du rapport entre la différence (Dq) et un pas prédéterminé d'incrément en débit (q) ;
- On détermine une valeur de compensation de pression (p[i]), à partir de cet indice (i) de débit respiratoire ;
- On détermine une valeur de pression de traitement en mode « Calibration Confort » (Pconfort) en fonction de la valeur de compensation de pression (p[i]) et de la valeur de la pression de traitement (Pconsigne), de façon à définir un mode « Calibration Confort ».

Ainsi, un tel procédé permet de surcompenser ou sous-compenser la pression délivrée au patient selon la phase respiratoire tout en conservant toute l'efficacité du traitement, quel que soit le traitement préconisé.

La pression de traitement (Pconsigne) peut être une pression constante correspondant à un traitement CPAP, ou une pression constante sur deux valeurs (selon que le patient est en phase d'inspiration ou d'expiration) correspondant à un traitement bi-level, ou une pression variable au cours du traitement correspondant à un traitement Auto-CPAP.

Ainsi, l'activation d'un mode « Calibration Confort » permet de générer une compensation de pression (p[i]) en plus de la pression de traitement (Pconsigne) de sorte que la pression en mode « Calibration Confort », c'est-à-dire (Pconfort), suive les variations de respiration du patient. La pression délivrée par l'appareil augmente pendant l'inspiration et diminue pendant l'expiration ce qui permet de limiter le sentiment de gêne du patient lorsqu'il expire, sans dégrader l'efficacité du traitement.

De préférence, l'on calcule l'indice (i) de débit respiratoire de la façon suivante :

- En mode CPAP ou Auto-CPAP (pression constante sur l'ensemble de la respiration) :

$$i = ((Qt - Qb) + e) / q$$

où : (Qt) est le débit total d'air délivré par l'appareil, (Qb) correspond aux valeurs de débits de fuites existantes dans le circuit patient, (e) est un décalage, de préférence fixé préalablement permettant de déterminer des indices (i) uniquement positifs ce qui facilite notamment la recherche d'indice lorsqu'une table préenregistrée est utilisée, et (q) est un pas d'incrément en débit préfixé.

- En mode Bi-level (pression (Pinsp) en phase d'inspiration et (Pexp) en phase d'expiration) :

$$i = (Qt - K\sqrt{Pmask(estimée)}) / q$$

où : (Qt) est le débit total d'air délivré par l'appareil, (Pmask-estimée) est la pression estimée au niveau du masque du patient, (K) est un coefficient déterminé en fin de phase expiratoire précédente selon l'équation $K = Qt/\sqrt{Pmask(estimée)}$, comme il l'est décrit dans la demande de brevet US2008/0097234, et (q) est un pas d'incrément en débit préfixé.

En effet, en mode Bi-level, les variations de niveau de pression délivrée lors des phases d'inspiration ou d'expiration ne permettent pas de déterminer simplement le débit respiratoire du patient à l'aide d'une valeur de base du débit total (Qb) correspondant aux valeurs de débit de fuites existantes dans le circuit patient. Il est donc nécessaire de prendre en compte dans le débit total (Qt) mesuré la variation de débit inhérente aux changements de pression entre les phases d'inspiration et les phases d'expiration, et la présence de fuites (volontaires et involontaires) dans le circuit d'air au patient.

La pression (Pconfort) est alors déterminée par :

$$(Pconfort) = (Pconsigne) + (p[i])$$

De préférence, l'on détermine au moins une valeur de compensation de pression (p[i]) à partir de l'indice (i) de débit respiratoire grâce à au moins une table de valeur.

En effet, les valeurs de compensation de pression (p[i]) ainsi que les indices (i) peuvent être stockés dans une table mémorisée.

Un tel procédé basé sur l'utilisation d'une table indicée permet une meilleure adaptabilité quel que soit l'appareil utilisé, et évite des lenteurs pouvant être dues aux temps de calcul, comparativement à l'utilisation d'une équation (par exemple polynomiale de degré élevé) et de minimiser les temps de calculs et l'utilisation du calculateur.

La table de coefficient est alors enregistrée sur une mémoire de l'appareil. Cette étape de pré-enregistrement a avantageusement lieu avant toute mise à disposition de l'appareil à un praticien.

Il est toutefois aussi possible de déterminer au moins une valeur de compensation de pression (p[i]) à partir de l'indice (i) de débit respiratoire grâce à au moins une équation.

Ceci permet de procurer davantage de flexibilité de maintenance et de possibilités d'évolution. Les tables de valeurs de compensation de pression sont ainsi plus simples et plus rapides à établir.

Par exemple, il est aussi possible de réguler cette valeur de pression (Pconfort) au niveau du masque du patient selon un principe similaire à celui de compensation des variations de pression inhérentes aux résistances pneumatiques du circuit, au débit de respiration du patient et aux débits de fuite par l'équation citée précédemment :

$$(Pmask\ estimée) = (Pout) - (Ai * Qt + Bi)$$

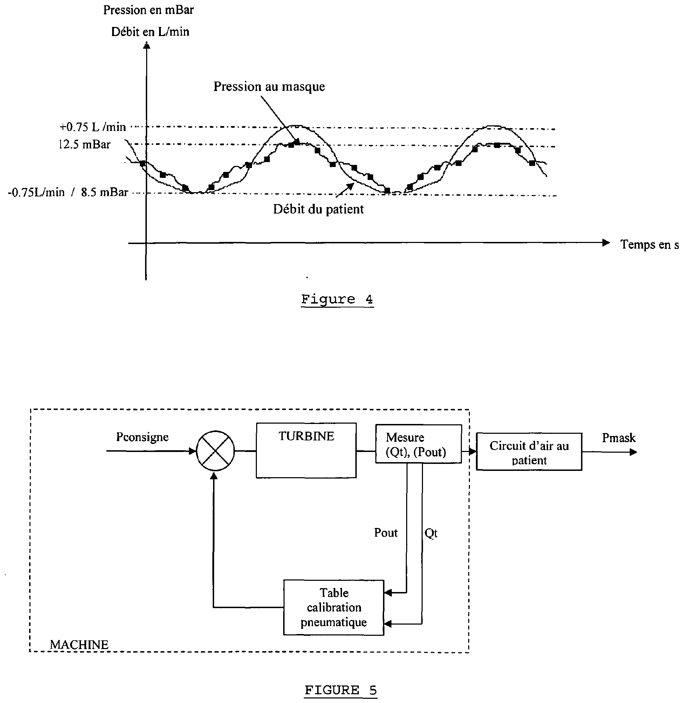

Le but étant d'avoir la pression (Pmask estimée) égale à la valeur de la pression (Pconsigne) au travers du système de régulation de pression, (Pconsigne=Pmask estimée)(Fig 5).

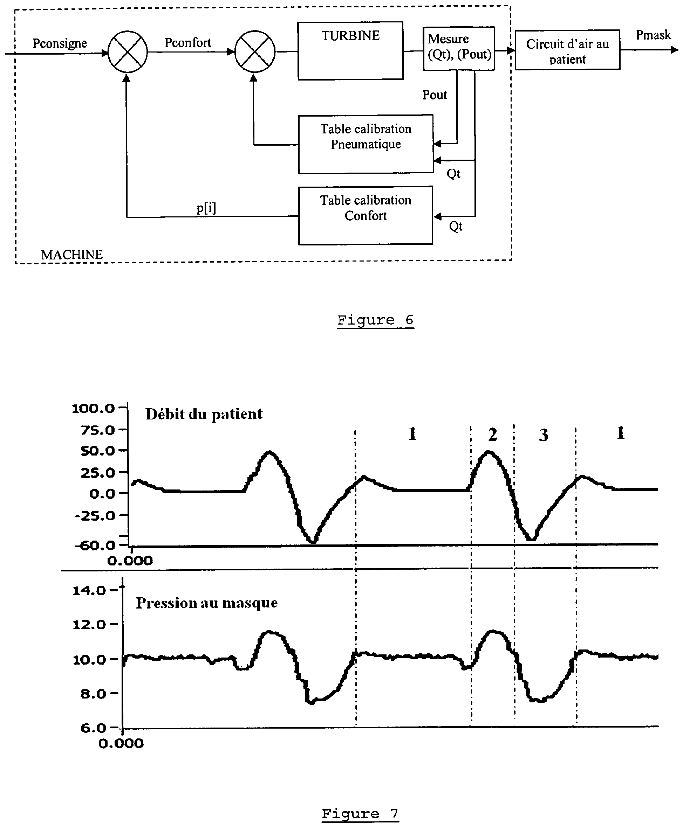

En appliquant le même procédé à la détermination de la pression de confort, on obtiendrait (Fig 6) :

Pconfort = (Pconsigne) + p[i]= (Pmask estimée) + p[i] Où (p[i]) est une fonction de (i) du type :

$$p[i]= A'[i] * (Qt-Qb) + B'[i]$$

où A'[i] et B'[i] sont des valeurs de coefficients déterminés empiriquement permettant la détermination de la pression de confort par approximation linéaire pour différentes plages de débit (Qt-Qb).

De préférence, le procédé comporte une étape préalable de paramétrage de l'appareil de traitement des troubles respiratoires du sommeil durant laquelle un usager enregistre au moins une valeur de (Pconsigne) et sélectionne une table de « Calibration Confort » à utiliser.

Un tel procédé requiert ainsi peu de paramétrage tout en s'adaptant à la majorité des patients.

Avantageusement, l'usager peut être aussi bien un praticien ayant prescrit le traitement que le patient lui-même. Par exemple, le faible nombre de paramètres ajustables facilite la mise en œuvre d'un tel procédé par le patient à qui le praticien aurait, de préférence, communiqué la valeur des paramètres.

De ce fait, il est possible de rendre le traitement plus confortable pour le patient en fonction de son ressenti, et plus souple d'utilisation.

Avantageusement, l'invention comporte également un mode « Auto Calibration Confort » afin de proposer aux patients présentant des amplitudes respiratoires différentes le même degré de confort. Dans ce mode « Auto Calibration Confort » le pas d'incrément en débit (q) est désormais déterminé en fonction de l'amplitude respiratoire du patient, alors qu'il était préfixé dans le mode « Calibration Confort » précédemment décrit. Par exemple, (q) est calculé de la façon suivante :

$$q = (Dq\_max - Dq\_min / (ni-1))$$

avec (ni) le nombre d'indices (i), Dq_max la valeur maximale de (Qt-Qb), Dq_min la valeur minimale de (Qt-Qb), et (Dq max-Dq min) représentant l'amplitude respiratoire du patient.

Cette amplitude respiratoire du patient peut être mesurée, par exemple, sur un certain nombre déterminé de cycles respiratoires (un cycle comportant une inspiration et une expiration) ou sur une durée prédéterminée.

Dans ce cas, le procédé comporte une étape supplémentaire consistant à mesurer l'amplitude respiratoire du patient sur un nombre prédéterminé de cycles, par exemple 8 cycles, puis à calculer le pas d'incrément en débit (q).

Une mesure de 8 cycles respiratoires est suffisante pour connaître l'amplitude de respiration moyenne d'un patient. Un plus grand nombre de cycles n'apporte pas davantage d'informations pertinentes à ce sujet, et un nombre trop faible n'est pas représentatif.

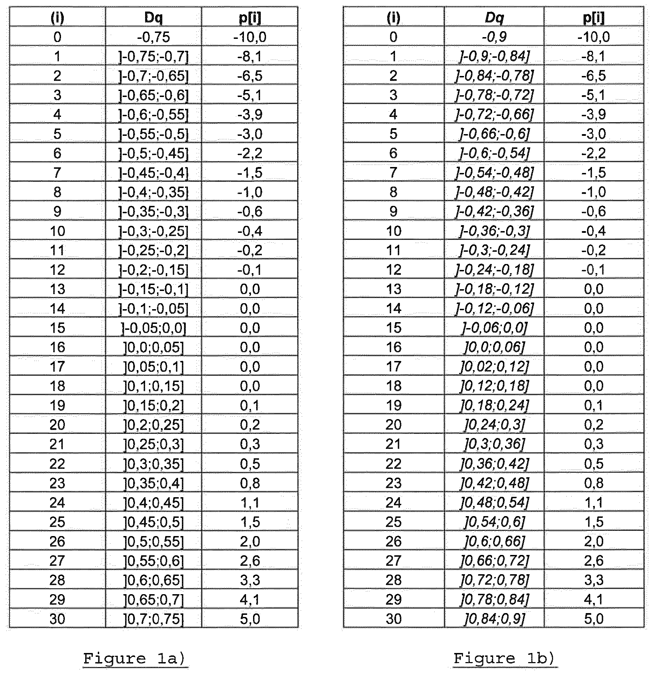

Une mesure au cours de 8 cycles peut intervenir à n'importe quel moment du traitement, ou au début de la mise en route de l'appareil de traitement. Ainsi, une table de « Calibration Confort » est échelonnée sur toute l'amplitude de respiration du patient, ce qui permet de compenser la pression avec la même régularité et le même degré de confort aussi bien pour des patients présentant de grandes amplitudes respiratoire que de petites amplitudes respiratoires. Par exemple, la table de la figure 1a) comprend 31 indices avec un pas d'incrément en débit (q) de 0,05 L/min, ce qui, pour un patient « standard », échelonne la table pour des valeurs de (Dq=Qt-Qb) comprises entre -0,75 L/min et +0,75 L/min, correspondant aux valeurs de compensation de pression de -10 dixièmes de mbars à 5 dixièmes de mbars respectivement. Dans le cas d'un patient présentant une plus grande amplitude respiratoire, le débit de base reste identique pour tout patient, mais le débit total est plus important, les valeurs de (Dq) varieraient, par exemple, de -0,90 L/min à +0,90 L/min. Puisque la table est fixée pour 31 valeurs avec des valeurs de compensation de pression de -10 dixièmes de mbars à 5 dixièmes de mbars respectivement pour procurer le même confort, le pas d'incrément en débit est alors de 0,06 L/min (Cf. figure 1b).

L'appareil de traitement des troubles respiratoires du sommeil d'un patient selon l'invention permet la mise en œuvre d'un procédé tel que défini précédemment. L'appareil comprend entre autres un générateur de flux d'air, un contrôleur du générateur, et un moyen d'activation du mode « Calibration Confort » commandant le contrôleur du générateur de sorte que le générateur délivre un flux d'air à une pression (Pconfort) déterminée par :

$$Pconfort = Pconsigne + p[i]$$

où :

(Pconfort) est la pression délivrée au patient lorsque le mode « Calibration Confort » est activé,

(Pconsigne) est la pression délivrée au patient lorsque le mode « Calibration Confort » n'est pas activé, c'est-à-dire la pression de traitement,

(p[i]) est la valeur de compensation de pression correspondant à l'indice (i) de débit respiratoire.

Cette valeur de compensation de pression (p[i]), une fois déterminée, est utilisée par le contrôleur du générateur, tel qu'une turbine, grâce à un processeur pour délivrer au patient la pression (Pconfort) de sorte à lui apporter une sensation améliorée de confort.

De préférence, l'appareil comprend une mémoire permettant de préenregistrer au moins une table de valeurs de compensation de pression (p[i]) en fonction de l'indice (i) de débit respiratoire.

La compensation de pression (p[i]) est ainsi directement connue en fonction de l'indice (i) de débit respiratoire calculé.

De préférence, l'appareil comprend au moins un algorithme de calcul pour calculer au moins une valeur de compensation de pression (p[i]) à partir de l'indice (i) de débit respiratoire.

Une ou plusieurs tables ou une ou plusieurs équations peuvent être stockées en mémoire puis utilisées pour modifier la pression appliquée au patient.

L'appareil comprend au moins un capteur de débit et de pression disposé dans le flux d'air avant une sortie d'air de l'appareil.

La présence d'un capteur est nécessaire dans le cas où, avantageusement, la valeur de pression (Pconfort) est régulée au niveau du masque du patient selon le même principe que pour compenser les variations de pression inhérentes aux résistances pneumatiques du circuit, aux débits de fuite, et aux débits de respiration du patient.

De préférence, l'appareil comprend au moins un processeur permettant de discrétiser un signal émis par au moins un capteur.

L'invention, selon un mode préférentiel de réalisation, sera bien comprise et ses avantages apparaîtront mieux à la lecture de l'exemple qui suit, à titre indicatif et nullement limitatif, et en référence aux dessins annexés présentés ci-après :

- La figure la), est un exemple d'une table de valeurs de compensation de pression échelonné pour un patient « standard », et la figure 1b) est échelonné pour un patient présentant une plus grande amplitude respiratoire ;

- La figure 2 est un graphe représentant le tracé de la table de la figure 1a), c'est-à-dire la pression de compensation (p[i]) en fonction de l'indice (i) correspond à une plage de débits (Dq) ;

- La figure 3 est un graphe représentant le débit d'air de patient et la pression délivrée par l'appareil en mode de traitement CPAP ;

- La figure 4 est un graphe représentant le débit d'air de patient et la pression délivrée par l'appareil en mode de traitement CPAP lorsque le mode « Calibration Confort » est activé ;

- La figure 5 est un schéma fonctionnel représentant le fonctionnement de la « Calibration pneumatique » dans un appareil, conformément à l'art antérieur.

- La figure 6 est un schéma fonctionnel représentant le fonctionnement de la « Calibration pneumatique et la « Calibration Confort », conformément à la présente invention.

- La figure 7 présente un exemple de variation de pression délivrée au masque en fonction du débit du patient.

- La figure 8 illustre le comportement de l'appareil lorsque le mode « Calibration Confort » est activé lors d'une pause respiratoire.

Une table contenant des valeurs de compensation de pression (p[i]) prédéterminées est stockée dans une mémoire d'un appareil d'assistance respiratoire.

La table de la figure 1a) comprend 31 valeurs. Les valeurs de compensation de pression (p[i]) correspondant aux indices (i) numérotés de 0 à 13 correspondent aux différentes pressions à appliquer à une pression de traitement (Pconsigne) lors des débits patients négatifs (phases d'expiration), alors que les valeurs de compensation de pression correspondant aux indices (i) numérotés de 18 à 30 correspondent aux différentes pression à appliquer à la pression de traitement (Pconsigne) lors des débits patients positifs (phases d'inspiration). Les indices (i=13) à (i=18) correspondent à la transition entre débit positifs et débits négatifs du patient. A ce moment, la pression délivrée au patient est donc la pression de traitement (Pconsigne), permettant ainsi de garantir un traitement efficace du patient.

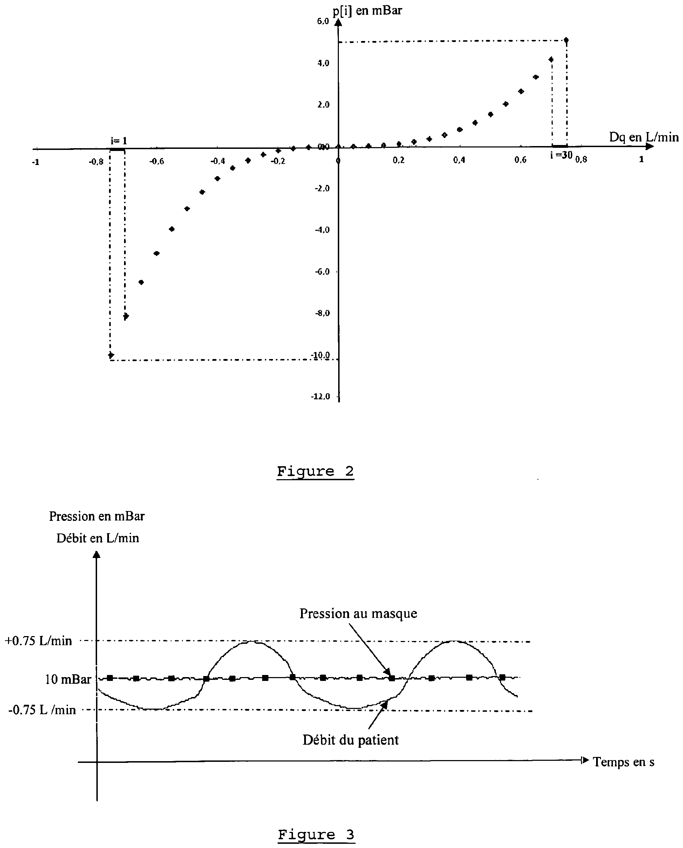

La figure 2 donne une représentation graphique de la table de la figure 1a). L'abscisse représente des différences de débit (Dq) (en litres par minute, L/min), et l'ordonnée représente la valeur de compensation de pression (p[i]) (en millibars, mBar) à ajouter à la valeur de la pression de traitement (Pconsigne) lorsque le mode « Calibration Confort » est activé.

La différence de débit (Dq) correspond à chaque instant à la différence entre un débit total (Qt) délivré par l'appareil et une valeur de base du débit total (Qb), qui en mode CPAP correspond aux valeurs de débit de fuites existantes dans le circuit patient.

Lors du traitement d'un patient, à chaque instant, à intervalles de temps (dt) réguliers, avec (dt) inférieur à 1 seconde, voire (dt) = 0,01 seconde, le débit total (Qt) est mesuré par un capteur, de préférence au niveau de la sortie de l'appareil, ainsi que la pression en sortie de l'appareil (Pout). Le signal du capteur, i.e. sa mesure, est discrétisé grâce à un processeur intégré dans l'appareil. Ainsi, à un instant (t), une valeur du débit total (Qt) est enregistrée.

Selon le présent exemple de réalisation, la « Calibration Confort » a lieu en parallèle d'une calibration pneumatique permettant de compenser des débits de fuite survenant le long du circuit d'air.

Pour la mise en œuvre de la « Calibration Confort », à chaque instant (t), on calcule alors l'indice (i), par exemple de la façon suivante en mode CPAP :

$$i = ((Qt - Qb) + e) / q.$$

Où (Qb) est la ligne de base du débit total (Qt) à l'instant (t), (e) un décalage permettant de n'obtenir que des valeurs d'indices (i) positives, et (q) un pas d'incrémentation prédéterminé en débit.

La valeur de compensation de pression (p[i]) est ainsi déterminée en temps réel. En fonction du résultat du calcul de (i), la valeur de compensation de pression (p[i]) est connue grâce à la table de valeur de la figure 1a), et la valeur de la pression délivrée au patient en mode « Calibration Confort » (Pconfort) est alors déterminée de la façon suivante :

$$Pconfort = Pconsigne + p[i]$$

A chaque instant (t), le processeur envoie ainsi une instruction au contrôleur du générateur pour que le générateur de flux d'air (en général, une turbine) adapte son régime de fonctionnement pour délivrer la pression à la valeur (Pconfort) ainsi déterminée.

Une boucle d'asservissement supplémentaire peut alors permettre de s'assurer que la pression délivrée au niveau du masque (Pmask) est bien celle visée.

Les figures 3 et 4 permettent de comparer l'évolution de la pression délivrée par l'appareil (une machine de traitement CPAP comprenant un humidificateur, pour un circuit pneumatique comprenant un conduit patient d'un diamètre de 22 millimètres et d'une longueur d'1,80 mètres) avec sa calibration « standard » correspondant à cette configuration, et cette même machine avec une « Calibration Confort ».

La table de la figure 1a) a été utilisée pour déterminer la variation de pression (P[i]) à appliquer à la pression de consigne (Pconsigne) délivrée par l'appareil en fonction du débit d'air du patient.

La machine est réglée pour délivrer une pression de traitement (Pconsigne) à environ 9,806 millibars (ce qui correspond à une pression de 10 centimètres d'eau, généralement noté 10cmH2O).

Sans le mode « Calibration Confort » (figure 3), la pression est maintenue constante à cette valeur tout au long du traitement par le dispositif conforme à l'art antérieur (i.e. juste avec une calibration pneumatique). Le patient éprouve alors un sentiment désagréable lors de l'expiration dû à une sensation de résistance du fait de la pression délivrée par la machine.

Lorsque le mode « Calibration Confort » est activé, la pression lors de l'expiration diminue et le patient ressent moins de difficulté à expirer. Lors de l'inspiration, la pression augmente procurant ainsi une sensation de facilité à inspirer pour le patient.

Lorsque le débit du patient est nul, la pression au masque est la pression de traitement prescrite.

Ce procédé permet d'utiliser des tables qui ont été judicieusement prédéfinies pour réagir de manière fiable autour des transitions, entre débit positif et négatif du patient, mais également lors d'événements respiratoires type apnée, afin de fournir lors de ces événements particuliers, la pression de traitement prescrite par le médecin.

La figure 7 permet d'illustrer l'évolution de la pression délivrée au masque (Pmask) lorsque le mode « Calibration Confort » est activé, au cours des différentes phases respiratoires du patient.

L'appareil, de même que précédemment, est réglé en mode CPAP avec une pression de consigne à (Pconsigne=10cmH2O), comprend un humidificateur, et un circuit pneumatique constitué d'un conduit patient d'un diamètre de 22 millimètres et d'une longueur d'1,80 mètres. Le mode « Calibration Confort » est activé.

Les mesures ont été effectuées avec un simulateur ASL5000, de volume 700 ml (millilitres), et une pression mesurée sur une fuite de 4 millimètres de diamètre.

Sur la figure 7, le premier graphe retrace le débit d'air délivré par le patient (en litres par minute (L/min) au cours du temps, ce qui montre son activité respiratoire, et le deuxième graphe présente l'évolution de la pression délivrée au masque (en centimètres d'eau, cmH2O) au cours de la même échelle de temps.

Une phase n°1 correspond à une phase transitoire lors de la respiration du patient, c'est-à-dire entre une expiration et une inspiration. Une phase n°2 correspond à une phase d'inspiration, et enfin, une phase n°3 à une phase d'expiration.

En phase transitoire (n°1), la pression délivrée au maque (Pmask) est bien égale la pression de consigne (Pconsigne) (les variations inhérentes aux différentes pertes de débits sont compensées grâce aux tables de calibration pneumatique, conformément à l'art antérieur). On peut donc lire sur le deuxième graphe de la figure 7 que la pression (Pmask) en phase transitoire est de 10cmH20. Lors de la phase inspiratoire (n°2), la pression au masque (Pmask) augmente d'environ 1.5cmH20, la pression au masque (Pmask) chute d'environ 2.5cmH20, et cette même pression se rétablit à 10cmH2O à la fin de l'expiration et durant toute la phase transitoire (1) .

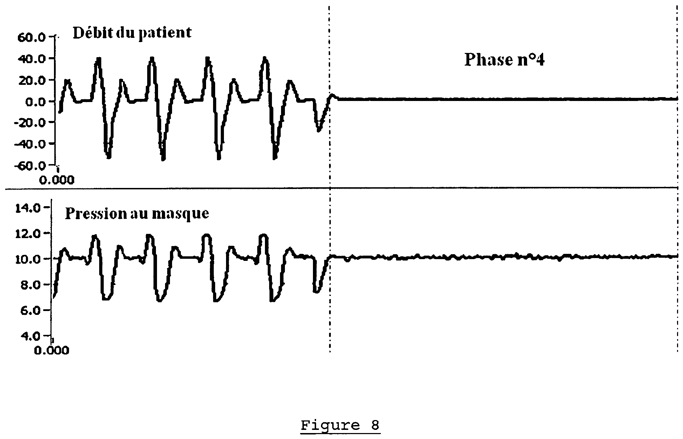

La figure 8 montre la mise en œuvre du procédé lors d'une phase d'apnée du patient, notée phase n°4.

De même que sur la figure 7, le premier graphe de la figure 8 retrace le débit d'air délivré par le patient (en litres par minute (L/min) au cours du temps, et le deuxième graphe présente l'évolution de la pression délivrée au masque (en centimètres d'eau, cmH2O) au cours de la même échelle de temps.

Ainsi, lorsque le patient entre dans une phase d'apnée, la pression délivrée par l'appareil est la pression de consigne (Pconsigne), préfixée à 10cmH2O.

## Revendications

1. Appareil de traitement des troubles respiratoires du sommeil configuré pour délivrer une pression de traitement (Pconsigne) à une valeur prédéterminée à chaque instant au niveau d'un masque (Pmask) porté par un patient, ledit appareil comprenant au moins un générateur de flux d'air, un contrôleur du générateur, et un capteur de débit et de pression qui permet de mesurer le débit total du flux d'air sortant de l'appareil (Qt) ainsi que la pression en sortie de l'appareil (Pout), **caractérisé en ce que** l'appareil est configuré pour, à chaque instant :

   - calculer la différence (Dq) entre le débit total (Qt) et une valeur de base du débit total (Qb), la valeur de base du débit total (Qb) correspondant aux valeurs de débits de fuites existantes dans le circuit patient ;
   - déterminer un indice (i) de débit respiratoire à partir du rapport entre la différence (Dq) et un pas prédéterminé d'incrément en débit (q) ;
   - déterminer une valeur de compensation de pression (p[i]) à partir de l'indice (i) de débit respiratoire ; et **en ce que** l'appareil comprend en outre un moyen d'activation d'un mode « Calibration Confort » commandant le contrôleur du générateur de sorte que le générateur délivre un flux d'air à une pression (Pconfort) en fonction de la valeur de compensation de pression (p[i]) et de la valeur de la pression de traitement (Pconsigne) selon la formule :

$$Pconfort = Pconsigne + p[i]$$

   où :

   - (Pconfort) est la pression délivrée lorsque le mode « Calibration Confort » est activé,
   - (Pconsigne) est la pression délivrée lorsque le mode « Calibration Confort » n'est pas activé,
   - (p[i]) est la valeur de compensation de pression correspondant à l'indice (i) de débit respiratoire.

2. Appareil selon la revendication 1, **caractérisé en ce qu'**il comprend une mémoire permettant de préenregistrer au moins une table de valeurs de compensation de pression (p[i]) en fonction de l'indice (i) de débit respiratoire.

3. Appareil selon l'une quelconque des revendications 1 ou 2, **caractérisé en ce qu'**il comprend au moins un algorithme de calcul pour calculer au moins une valeur de compensation de pression (p[i]) à partir de l'indice (i) de débit respiratoire.

4. Appareil selon la revendication 3, **caractérisé en ce qu'**il comprend au moins un processeur permettant de discrétiser un signal émis par au moins le capteur.

5. Appareil selon la revendication 1 **caractérisé en ce qu'**il est configuré pour, lorsque la pression de consigne est constante sur l'ensemble de la respiration, calculer l'indice (i) de débit respiratoire de la façon suivante :

$$i = ((Qt - Qb) + e) / q$$

où :

(Qt) est le débit total d'air délivré par l'appareil,
(Qb) est la valeur de base du débit total correspondant aux valeurs de débits de fuites existantes dans le circuit patient,
(e) est un décalage, défini afin d'obtenir que des valeurs d'indices (i) positives,
(q) est le pas prédéterminé d'incrément en débit.

6. Appareil selon la revendication 1, **caractérisé en ce qu'**il est configuré pour, lorsque la pression est en mode Bi-level , calculer l'indice (i) de débit respiratoire de la façon suivante :

$$i = ((Qt - K\sqrt{Pmask(estimée)})) / q$$

où :

(Qt) est le débit total d'air délivré par l'appareil,
(K) est un coefficient déterminé en fin de phase expiratoire précédente selon l'équation K = Qt /√(P$_{mask(estimée)}$),
(Pmask estimée) est une pression estimée au niveau du masque, et
(q) est le pas prédéterminé d'incrément en débit.

7. Appareil selon l'une quelconque des revendications précédentes, **caractérisé en ce qu'**il est configuré pour mesurer l'amplitude du flux respiratoire sur un nombre prédéterminé de cycles, puis pour calculer le pas d'incrément en débit (q).

**Patentansprüche**

1. Vorrichtung zur Behandlung von Schlafatmungsstörungen, die dazu ausgebildet ist, einen Behandlungsdruck (Pvorgabe) auf einem vorbestimmten Wert zu jedem Zeitpunkt an einer Maske (Pmask) zu liefern, die von einem Patienten getragen wird, wobei die Vorrichtung wenigstens einen Luftstromgenerator, eine Steuerung des Generators und einen Strömungsraten- und Drucksensor, der es ermöglicht, die Gesamtströmungsrate des Luftstroms, der aus der Vorrichtung austritt (Qt), sowie den Druck am Ausgang der Vorrichtung (Pout) zu messen, umfasst, **dadurch gekennzeichnet, dass** die Vorrichtung dazu ausgebildet ist, zu jedem Zeitpunkt:

- die Differenz (Dq) zwischen der Gesamtströmungsrate (Qt) und einem Grundwert der Gesamtströmungsrate (Qb) zu berechnen, wobei der Grundwert der Gesamtströmungsrate (Qb) den Werten von Strömungsraten von Lecks entspricht, die im Patientenkreislauf vorhanden sind;
- einen Atemleistungsindex (i) ausgehend von dem Verhältnis zwischen der Differenz (Dq) und einem vorbestimmten Strömungsraten-Inkrementabstand (q) zu bestimmen;
- einen Druckausgleichswert (p[i]) ausgehend von dem Atemleistungsindex (i) zu bestimmen;
und dadurch, dass die Vorrichtung ferner ein Mittel zur Aktivierung eines "Komfortkalibierungs-" Modus umfasst, der die Steuerung des Generators so steuert, dass der Generator einen Luftstrom mit einem Druck (Pconfort) in Abhängigkeit vom Druckausgleichswert (p[i]) und vom Wert des Behandlungsdrucks (Pvorgabe) gemäß der folgenden Formel liefert:

$$Pconfort = Pvorgabe + p[i]$$

wobei:

- (Pconfort) der Druck ist, der geliefert wird, wenn der "Komfortkalibierungs-" Modus aktiviert ist,
- (Pvorgabe) der Druck ist, der geliefert wird, wenn der "Komfortkalibierungs-" Modus nicht aktiviert ist,
- (p[i]) der Druckausgleichswert ist, der dem Atemleistungsindex (i) entspricht.

2. Vorrichtung nach Anspruch 1, **dadurch gekennzeichnet, dass** sie einen Speicher umfasst, der es ermöglicht, wenigstens eine Tabelle mit Druckausgleichswerten (p[i]) in Abhängigkeit von dem Atemleistungsindex (i) vorab zu speichern.

3. Vorrichtung nach einem der Ansprüche 1 oder 2, **dadurch gekennzeichnet, dass** sie wenigstens einen Rechenalgorithmus zur Berechnung wenigstens eines Druckausgleichswerts (p[i]) ausgehend von dem Atemleistungsindex (i) umfasst.

4. Vorrichtung nach Anspruch 3, **dadurch gekennzeichnet, dass** sie wenigstens einen Prozessor umfasst, der es ermöglicht, ein Signal zu diskretisieren, das wenigstens von dem Sensor abgegeben wird.

5. Vorrichtung nach Anspruch 1, **dadurch gekennzeichnet, dass** sie dazu ausgebildet ist, wenn der Vorgabedruck über die gesamte Atmung konstant ist, den Atemleistungsindex (i) auf folgende Weise zu berechnen:

$$i = ( (Qt - Qb) + e) / q$$

wobei:

(Qt) die Gesamtströmungsrate der Luft ist, die von der Vorrichtung geliefert wird,
(Qb) der Grundwert der Gesamtströmungsrate ist, der den Werten von Strömungsraten von Lecks entspricht, die im Patientenkreislauf vorhanden sind,
(e) ein Versatz ist, der festgelegt ist, um nur positive Indexwerte (i) zu erhalten,
(q) der vorbestimmte Strömungsraten-Inkrementabstand ist.

6. Vorrichtung nach Anspruch 1, **dadurch gekennzeichnet, dass** sie dazu ausgebildet ist, wenn sich der Druck im Bi-Level-Modus befindet, den Atemleistungsindex (i) auf folgende Weise zu berechnen:

$$i = \left( \left( Qt - K \sqrt{Pmask \text{ (geschätzt)}} \right) \right) / q$$

wobei: (Qt) die Gesamtströmungsrate der Luft ist, die von der Vorrichtung geliefert wird,

(K) ein Koeffizient ist, der am Ende einer vorangehenden Ausatmungsphase gemäß der Gleichung

$$K = Qt / \sqrt{(P_{mask\,(geschätzt)})}$$ bestimmt wird,

(Pmask geschätzt) ein geschätzter Druck an der Maske ist und
(q) der vorbestimmte Strömungsraten-Inkrementabstand ist.

7. Vorrichtung nach einem der vorhergehenden Ansprüche, **dadurch gekennzeichnet, dass** sie dazu ausgebildet ist, die Amplitude der Atemströmung über eine vorbestimmte Anzahl von Zyklen zu messen, dann den Strömungsraten-Inkrementabstand (q) zu berechnen.

**Claims**

1. Apparatus for treating sleep-related respiratory disorders, configured to deliver a treatment pressure (Psetpoint) at a predetermined value at each instant to a mask (Pmask) worn by a patient, said apparatus comprising at least one air flow generator, a controller of the generator, and a flowrate and pressure sensor which makes it possible to measure the total flowrate of the air flow issuing from the apparatus (Qt) and also the outlet pressure of the apparatus (Pout), **characterized in that** the apparatus is configured to at each instant:

- calculate the difference (Dq) between the total flowrate (Qt) and a base value (Qb) of the total flowrate, the base value (Qb) of the total flowrate corresponding to the flowrate values of leaks existing in the patient circuit;
- determine a respiratory flowrate index (i) from the ratio between the difference (Dq) and a predetermined step of increment in flowrate (q);
- determine a pressure compensation value (p[i]) from the respiratory flowrate index (i);
and **in that** the apparatus additionally comprises a means of activation of a "Comfort Calibration" mode commanding the controller of the generator such that the generator delivers an air flow at a pressure (Pcomfort) as a function of the pressure compensation value (p[i]) and of the value of the treatment pressure (Psetpoint) according to the formula:

$$Pcomfort = Psetpoint + p[i]$$

where:

- (Pcomfort) is the pressure delivered when the "Comfort Calibration" mode is activated,
- (Psetpoint) is the pressure delivered when the "Comfort Calibration" mode is not activated,
- (p[i]) is the pressure compensation value corresponding to the respiratory flowrate index (i).

2. Apparatus according to Claim 1, **characterized in that** it comprises a memory for pre-registering at least one table of pressure compensation values (p[i]) as a function of the respiratory flowrate index (i).

3. Apparatus according to either of Claims 1 and 2, **characterized in that** it comprises at least one calculation algorithm for calculating at least one pressure compensation value (p[i]) from the respiratory flowrate index (i).

4. Apparatus according to Claim 3, **characterized in that** it comprises at least one processor making it possible to discretize a signal emitted by at least the sensor.

5. Apparatus according to Claim 1, **characterized in that** it is configured such that, with the setpoint pressure constant across the entire respiration, it calculates the respiratory flowrate index (i) in the following way:

$$i = ((Qt - Qb) + e) / q$$

where:

(Qt) is the total flowrate of air delivered by the apparatus,
(Qb) is the base value of the total flowrate corresponding to the values of flowrates of leaks existing in the patient circuit,
(e) is an offset defined so as to obtain only positive index values (i),
(q) is the predetermined step of increment in flowrate.

6. Apparatus according to Claim 1, **characterized in that** it is configured such that, with the pressure in bi-level mode, it calculates the respiratory flowrate index (i) in the following way:

$$i = ((Qt - K = \sqrt{Pmask}\ (estimated)) / q$$

where:

(K) is a coefficient determined at the end of the preceding expiratory phase according to the equation $K = Qt / \sqrt{P}_{mask(estimated)})$,
(Pmask estimated) is a pressure estimated at the mask, and
(q) is the predetermined step of increment in flowrate.

7. Apparatus according to any one of the preceding claims, **characterized in that** it is configured to measure the amplitude of the respiratory flow over a predetermined number of cycles, then to calculate the step of increment in flowrate (q).

| (i) | Dq | p[i] |
|-----|------|------|
| 0 | -0,75 | -10,0 |
| 1 | ]-0,75;-0,7] | -8,1 |
| 2 | ]-0,7;-0,65] | -6,5 |
| 3 | ]-0,65;-0,6] | -5,1 |
| 4 | ]-0,6;-0,55] | -3,9 |
| 5 | ]-0,55;-0,5] | -3,0 |
| 6 | ]-0,5;-0,45] | -2,2 |
| 7 | ]-0,45;-0,4] | -1,5 |
| 8 | ]-0,4;-0,35] | -1,0 |
| 9 | ]-0,35;-0,3] | -0,6 |
| 10 | ]-0,3;-0,25] | -0,4 |
| 11 | ]-0,25;-0,2] | -0,2 |
| 12 | ]-0,2;-0,15] | -0,1 |
| 13 | ]-0,15;-0,1] | 0,0 |
| 14 | ]-0,1;-0,05] | 0,0 |
| 15 | ]-0,05;0,0] | 0,0 |
| 16 | ]0,0;0,05] | 0,0 |
| 17 | ]0,05;0,1] | 0,0 |
| 18 | ]0,1;0,15] | 0,0 |
| 19 | ]0,15;0,2] | 0,1 |
| 20 | ]0,2;0,25] | 0,2 |
| 21 | ]0,25;0,3] | 0,3 |
| 22 | ]0,3;0,35] | 0,5 |
| 23 | ]0,35;0,4] | 0,8 |
| 24 | ]0,4;0,45] | 1,1 |
| 25 | ]0,45;0,5] | 1,5 |
| 26 | ]0,5;0,55] | 2,0 |
| 27 | ]0,55;0,6] | 2,6 |
| 28 | ]0,6;0,65] | 3,3 |
| 29 | ]0,65;0,7] | 4,1 |
| 30 | ]0,7;0,75] | 5,0 |

Figure 1a)

| (i) | Dq | p[i] |
|-----|------|------|
| 0 | -0,9 | -10,0 |
| 1 | ]-0,9;-0,84] | -8,1 |
| 2 | ]-0,84;-0,78] | -6,5 |
| 3 | ]-0,78;-0,72] | -5,1 |
| 4 | ]-0,72;-0,66] | -3,9 |
| 5 | ]-0,66;-0,6] | -3,0 |
| 6 | ]-0,6;-0,54] | -2,2 |
| 7 | ]-0,54;-0,48] | -1,5 |
| 8 | ]-0,48;-0,42] | -1,0 |
| 9 | ]-0,42;-0,36] | -0,6 |
| 10 | ]-0,36;-0,3] | -0,4 |
| 11 | ]-0,3;-0,24] | -0,2 |
| 12 | ]-0,24;-0,18] | -0,1 |
| 13 | ]-0,18;-0,12] | 0,0 |
| 14 | ]-0,12;-0,06] | 0,0 |
| 15 | ]-0,06;0,0] | 0,0 |
| 16 | ]0,0;0,06] | 0,0 |
| 17 | ]0,02;0,12] | 0,0 |
| 18 | ]0,12;0,18] | 0,0 |
| 19 | ]0,18;0,24] | 0,1 |
| 20 | ]0,24;0,3] | 0,2 |
| 21 | ]0,3;0,36] | 0,3 |
| 22 | ]0,36;0,42] | 0,5 |
| 23 | ]0,42;0,48] | 0,8 |
| 24 | ]0,48;0,54] | 1,1 |
| 25 | ]0,54;0,6] | 1,5 |
| 26 | ]0,6;0,66] | 2,0 |
| 27 | ]0,66;0,72] | 2,6 |
| 28 | ]0,72;0,78] | 3,3 |
| 29 | ]0,78;0,84] | 4,1 |
| 30 | ]0,84;0,9] | 5,0 |

Figure 1b)

Figure 2

Figure 3

Pression en mBar

Débit en L/min

Pression au masque

+0.75 L /min

12.5 mBar

-0.75L/min / 8.5 mBar

Débit du patient

Temps en s

Figure 4

MACHINE

Pconsigne

TURBINE

Mesure (Qt), (Pout)

Circuit d'air au patient

Pmask

Pout

Qt

Table calibration pneumatique

FIGURE 5

Figure 6

Figure 7

Figure 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20090241952 A1, de Nicolazzi
- WO 2008100859 A2
- WO 2004112680 A2
- US 2003121519 A1
- WO 2005070488 A1
- US 20080097234 A